# EUROPEAN PATENT SPECIFICATION

(11) **EP 4 313 267 B1**
(45) Date of publication and mention of the grant of the patent: **08.07.2026**
(21) Application number: 22715295.6
(22) Date of filing: 21.03.2022
(51) Int. Cl.: A61N 1/36, A61N 1/378, A61N 1/05

(54) **RECHARGEABLE SYSTEMS FOR DELIVERING ELECTRICAL STIMULATION THERAPY FOR CANCER THERAPY**
WIEDERAUFLADBARE SYSTEME ZUR VERABREICHUNG EINER ELEKTRISCHEN STIMULATIONSTHERAPIE FÜR DIE KREBSTHERAPIE
SYSTÈMES RECHARGEABLES POUR ADMINISTRER UNE THÉRAPIE PAR STIMULATION ÉLECTRIQUE POUR LA THÉRAPIE DU CANCER

(30) Priority: 22.03.2021 US 202163164445 P; 18.03.2022 US 202217698516
(43) Date of publication of application: 07.02.2024
(73) Proprietor: Cardiac Pacemakers, Inc., St. Paul, Minnesota 55112-5798 (US)
(72) Inventor: SCHMIDT, Brian L., White Bear Lake, Minnesota 55110 (US); ARNHOLT, Devon N., Shoreview, Minnesota 55126 (US); AVILES DELGADO, Emilio A., Toa Alta, 00953 (PR); COLLAZO RODRIGUEZ, David, Guaynabo, 00969 (PR)
(74) Representative: Vossius & Partner Patentanwälte Rechtsanwälte mbB
(86) International application number: PCT/US2022/021161
(87) International publication number: WO 2022/204035

(56) References cited:
- EP-A2- 2 497 530
- WO-A1-2017/044904
- US-A1- 2007 179 550
- US-A1- 2007 270 916
- US-A1- 2019 117 971

## Description

### Field

Embodiments herein relate to electrical stimulation-based cancer therapy systems. More specifically, embodiments herein relate to rechargeable electrical stimulation-based cancer therapy systems.

### Background

According to the American Cancer Society, cancer accounts for nearly 25% of the deaths that occur in the United States each year. The current standard of care for cancerous tumors can include first-line therapies such as surgery, radiation therapy, and chemotherapy. Additional second-line therapies can include radioactive seeding, cryotherapy, hormone or biologics therapy, ablation, and the like. Combinations of first-line therapies and second-line therapies can also be a benefit to patients if one particular therapy on its own is not effective.

Cancerous tumors can form if one normal cell in any part of the body mutates and then begins to grow and multiply too much and too quickly. Cancerous tumors can be a result of a genetic mutation to the cellular DNA or RNA that arises during cell division, an external stimulus such as ionizing or non-ionizing radiation, exposure to a carcinogen, or a result of a hereditary gene mutation. Regardless of the etiology, many cancerous tumors are the result of unchecked rapid cellular division.

Mitosis is the process of cellular division that is a part of the cell cycle for all somatic cells in the body, including many types of cancerous cells. Mitosis includes four basic phases: prophase, metaphase, anaphase, and telophase. Just prior to prophase, a cell will copy its chromosomes to create two identical sister chromatids. During prophase, the chromosomes start to condense and the nuclear membrane surrounding the nucleus disappears. The mitotic spindle also begins to form during prophase. The mitotic spindle includes a self-organized bipolar array of microtubules and centrosomes. Microtubules are generally formed from the polymerization of the highly polar alpha-tubulin and beta-tubulin proteins. Centrosomes are similarly protein-based organelles, two of which migrate to opposite sides of the dividing cell at this phase. The negatively charged end of the microtubules attach to the centrosomes. The positively charged end of the microtubules radiate toward the equator of the dividing cell where they eventually attach to a kinetochore of each sister chromatid. Metaphase can be defined by all chromosomes being aligned at the equator of the dividing cell and bound in the mitotic spindle. An equal number of sister chromatids are then pulled toward opposite ends of the cell during anaphase. Once all chromosomes have been separated, the process of telophase begins, where the cell membrane begins to form a cleavage furrow between the two newly forming sister cells, and cell division becomes complete once the cells physically separate from one another in a process called cytokinesis. WO 2017/044904 A1 discloses methods of treating a patient comprising providing a medical apparatus comprising an external system and an implantable system, implanting the implantable system, and delivering at least one of power or data to the implantable system with the external system. US2019117971A1 discloses volume-filling leads for treatment of cancer with electric fields.

### Summary

The invention is defined by the claims. In the following all methods of treatment are not claimed and are disclosed for illustrative purposes only.

Embodiments herein relate to rechargeable electrical stimulation-based cancer therapy systems and related methods. In a first aspect, an electrical stimulation-based cancer therapy system is included having an implantable electrical field generator unit including a housing, a header coupled to the housing, and electrical field generation circuitry is disposed within the housing. The system can also include an implantable recharge lead, wherein the implantable recharge lead is removably coupled to the header. The system can also include a plurality of therapy leads, the therapy leads including a plurality of electrodes, wherein the plurality of therapy leads area also removably coupled to the header. The system can also include an external recharger unit.

In a second aspect, in addition to one or more of the preceding or following aspects, or in the alternative to some aspects, the external recharger unit can include a recharger coil, a recharger lead, and a recharger control unit.

In a third aspect, in addition to one or more of the preceding or following aspects, or in the alternative to some aspects, the implantable recharge lead can include a connection plug, a recharge coil, and a recharge lead body, wherein the recharge lead body interconnects the connection plug and the recharge coil.

In a fourth aspect, in addition to one or more of the preceding or following aspects, or in the alternative to some aspects, the connection plug is configured to fit within the header.

In a fifth aspect, in addition to one or more of the preceding or following aspects, or in the alternative to some aspects, the connection plug can include a DF-1 terminal pin.

In a sixth aspect, in addition to one or more of the preceding or following aspects, or in the alternative to some aspects, the recharge lead body can include a guidewire lumen.

In a seventh aspect, in addition to one or more of the preceding or following aspects, or in the alternative to some aspects, the header can include a plurality of therapy lead receiving channels and one or more recharge lead receiving channels.

In an eighth aspect, in addition to one or more of the preceding or following aspects, or in the alternative to some aspects, the one or more recharge lead receiving channels are disposed on top of the plurality of therapy lead receiving channels.

In a ninth aspect, in addition to one or more of the preceding or following aspects, or in the alternative to some aspects, the housing can include a feed-through structure, and the housing can define a hermetically sealed interior volume. The header can include a plurality of conductors, wherein the plurality of conductors pass through the feed-through structure.

In a tenth aspect, in addition to one or more of the preceding or following aspects, or in the alternative to some aspects, the therapy leads can include at least two coil electrodes.

In an eleventh aspect, in addition to one or more of the preceding or following aspects, or in the alternative to some aspects, each of the at least two coil electrodes is from 0.5 cm to 4 cm in length.

In a twelfth aspect, in addition to one or more of the preceding or following aspects, or in the alternative to some aspects, each of the at least two coil electrodes is from 1.5 cm to 2.5 cm in length.

In a thirteenth aspect, in addition to one or more of the preceding or following aspects, or in the alternative to some aspects, each of the at least two coil electrodes has an outside diameter of 1 to 1.5 mm.

In a fourteenth aspect, in addition to one or more of the preceding or following aspects, or in the alternative to some aspects, each of the at least two coil electrodes has an outside diameter of 1.2 to 1.4 mm.

In a fifteenth aspect, in addition to one or more of the preceding or following aspects, or in the alternative to some aspects, the therapy leads can include a gap, wherein the gap is disposed between each of at least two coil electrodes, and wherein the gap is from 2 mm to 8 mm in length.

In a sixteenth aspect, in addition to one or more of the preceding or following aspects, or in the alternative to some aspects, the gap is from 4 mm to 6 mm in length.

In a seventeenth aspect, in addition to one or more of the preceding or following aspects, or in the alternative to some aspects, the therapy leads can include a guidewire lumen.

In an eighteenth aspect, in addition to one or more of the preceding or following aspects, or in the alternative to some aspects, the therapy leads can include a temperature sensor.

In a nineteenth aspect, in addition to one or more of the preceding or following aspects, or in the alternative to some aspects, the temperature sensor can include a thermistor.

In a twentieth aspect, a method of delivering electrical stimulation therapy to a patient is included. The method including implanting an implantable electric field generator unit, implanting a plurality of therapy leads, and implanting an implantable recharge lead. The method can also include connecting the plurality of therapy leads to the implantable electric field generator unit and connecting the implantable recharge lead to the implantable electric field generator unit.

In a twenty-first aspect, in addition to one or more of the preceding or following aspects, or in the alternative to some aspects, implanting the plurality of therapy leads is performed using a tunneling technique.

In a twenty-second aspect, in addition to one or more of the preceding or following aspects, or in the alternative to some aspects, the implantable electric field generator unit is implanted separately from the implantable recharge lead.

In a twenty-third aspect, in addition to one or more of the preceding or following aspects, or in the alternative to some aspects, implanting the implantable recharge lead is performed using a tunneling technique.

In a twenty-fourth aspect, in addition to one or more of the preceding or following aspects, or in the alternative to some aspects, the implantable recharge lead is removably coupled to a header.

In a twenty-fifth aspect, in addition to one or more of the preceding or following aspects, or in the alternative to some aspects, the therapy leads can include a plurality of electrodes.

In a twenty-sixth aspect, in addition to one or more of the preceding or following aspects, or in the alternative to some aspects, the electrodes can include at least two coil electrodes.

In a twenty-seventh aspect, in addition to one or more of the preceding or following aspects, or in the alternative to some aspects, each of the at least two coil electrodes is from 0.5 cm to 4 cm in length.

In a twenty-eighth aspect, in addition to one or more of the preceding or following aspects, or in the alternative to some aspects, each of the at least two coil electrodes has an outside diameter of 1.2 to 1.4 mm.

In a twenty-ninth aspect, in addition to one or more of the preceding or following aspects, or in the alternative to some aspects, the implantable electric field generator unit can include a housing and electrical field generation circuitry disposed within the housing, and a header coupled to the housing.

In a thirtieth aspect, in addition to one or more of the preceding or following aspects, or in the alternative to some aspects, the header can include a plurality of therapy lead receiving channels and one or more recharge lead receiving channels.

In a thirty-first aspect, in addition to one or more of the preceding or following aspects, or in the alternative to some aspects, the one or more recharge lead receiving channels are disposed on top of the plurality of therapy lead receiving channels.

In a thirty-second aspect, in addition to one or more of the preceding or following aspects, or in the alternative to some aspects, the method can further include fitting the patient with an external recharger unit.

In a thirty-third aspect, in addition to one or more of the preceding or following aspects, or in the alternative to some aspects, the external recharger unit can include a recharge control unit, a recharge lead, and a recharger coil.

In a thirty-fourth aspect, in addition to one or more of the preceding or following aspects, or in the alternative to some aspects, the method can further include tunneling a path through tissue between the implantable electric field generator unit and the implantable recharge lead, wherein the tunneled path is smaller than the recharger coil.

In a thirty-fifth aspect, in addition to one or more of the preceding or following aspects, or in the alternative to some aspects, the therapy leads can include a gap.

In a thirty-sixth aspect, in addition to one or more of the preceding or following aspects, or in the alternative to some aspects, the gap is disposed between each of at least two coil electrodes.

In a thirty-seventh aspect, in addition to one or more of the preceding or following aspects, or in the alternative to some aspects, the gap is from 0.5 cm to 3 cm in length.

In a thirty-eighth aspect, in addition to one or more of the preceding or following aspects, or in the alternative to some aspects, the method can further include administering electrical stimulation therapy as generated by the implantable electric field generator unit to the patient.

In a thirty-ninth aspect, in addition to one or more of the preceding or following aspects, or in the alternative to some aspects, the therapy leads can include a temperature sensor.

In a fortieth aspect, in addition to one or more of the preceding or following aspects, or in the alternative to some aspects, the temperature sensor can include a thermistor.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### Brief Description of the Figures

Aspects may be more completely understood in connection with the following figures (FIGS.), in which:
FIG. 1 is a schematic view of an electrical stimulation-based cancer therapy system in accordance with various embodiments herein.
FIG. 2 is a schematic view of implantable components of a system in accordance with various embodiments herein.
FIG. 3 is a schematic view of external components of a system in accordance with various embodiments herein.
FIG. 4 is a schematic view of a header in accordance with various embodiments herein.
FIG. 5 is a schematic view of a portion of a therapy lead in accordance with various embodiments herein.
FIG. 6 is a sectional view of a portion of a therapy lead in accordance with various embodiments herein.
FIG. 7 is a schematic view of a patient fitted with a system in accordance with various embodiments herein.
FIG. 8 is a schematic perspective view of therapy leads interfacing with a tumor in accordance with various embodiments herein.
FIG. 9 is a schematic plan view of therapy leads interfacing with a tumor in accordance with various embodiments herein.
FIG. 10 is a schematic view of a patient fitted with external components of a system in accordance with various embodiments herein.

While embodiments are susceptible to various modifications and alternative forms, specifics thereof have been shown by way of example and drawings, and will be described in detail. It should be understood, however, that the scope herein is not limited to the particular aspects described. On the contrary, the intention is to cover modifications, equivalents, and alternatives falling within the scope of the claims.

### Detailed Description

Embodiments herein include electrical stimulation-based cancer therapy systems. The system can delivery electrical stimulation therapy that is effective to interfere with the process of mitosis in cancerous cells. The system can include a set of implantable components such as an implantable electrical field generator unit that can include a housing, a header coupled to the housing, and electrical field generation circuitry disposed within the housing. The implantable components can also include an implantable recharge lead that is removably coupled to the header. The implantable components can also include a plurality of therapy leads, the therapy leads comprising a plurality of electrodes. The plurality of therapy leads can be removably coupled to the header. The system can include a set of external components including an external recharger unit. The external recharger unit can include a recharger coil, a recharge lead, and a recharge control unit.

While not intending to be bound by theory, the fact that the implantable recharge lead can be removably attached to the header of the implantable electric field generator unit offers substantial advantages for the implantation of the system. In specific, and as described further below, this allows for the formation of a tunnel through the tissue of the patient that is only as large as the implantable recharge lead body versus the much larger implantable recharge coil unit. Smaller tunnels are easier to make and can lead to faster healing times after implantation.

In addition, various embodiments herein provide for two or more coil electrodes on the stimulation leads having a length of 0.5 cm to 4 cm each with a gap in between at a distance of from 2 mm to 8 mm in length. While not intending to be bound by theory, this configuration has been found to be ideal for providing electrical stimulation at desired field strengths while providing for a desirable level of spatial diversity in the possible vectors across which stimulation can be provided.

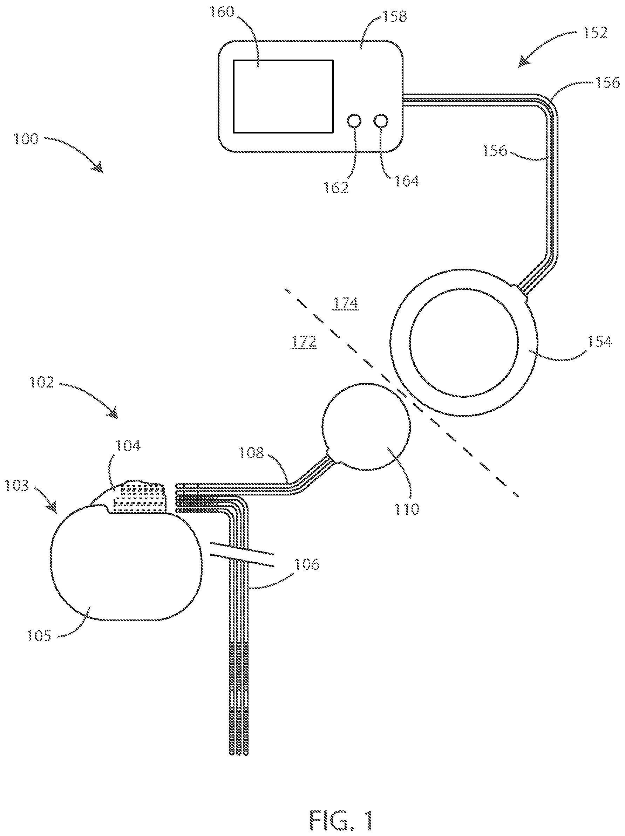

Referring now to FIG. 1, a schematic view of an electrical stimulation-based cancer therapy system 100 is shown in accordance with various embodiments herein. For context, FIG. 1 shows an inside 172 of the body of a patient along with an outside 174 the body of the patient, with the dividing line being the skin of the patient. The electrical stimulation-based cancer therapy system 100 includes implantable components 102 that can be implanted on the inside 172 of the body and external components 152 that can be used while remaining on the outside 174 of the body.

The implantable components 102 can include an implantable electrical field generator unit 103. The implantable electrical field generator unit 103 can serve to generate electrical fields for the treatment of tissue, such as cancerous tissue and/or tumors in accord with the therapy parameters described in greater detail below. The implantable components 102 can also include one or more therapy leads 106. The implantable components 102 can also include one or more implantable recharge leads 108. The implantable recharge lead 108 can include a recharge coil 110 (or recharge antenna).

The implantable electrical field generator unit 103 also includes a housing 105. The implantable electrical field generator unit 103 includes a header 104 that can be attached or otherwise coupled to the housing 105. The housing 105 can include one or more connection ports or channels in order to facilitate connecting the proximal ends of the therapy leads 106 and the recharge leads 108 thereto.

The therapy leads 106 and the recharge leads 108 can be removably attached or otherwise coupled to the implantable electrical field generator unit 103 via interface with the header 104 and, more specifically, the connection ports or channels thereof. In practice, a tunnel through tissue may be formed between the site where the implantable electrical field generator unit 103 is to be implanted and the site where the recharge coil 110 is to be implanted.

The external components 152 can include an external recharger unit that includes a recharger coil 154 (or recharger antenna). The external recharger unit can also include a recharger lead 156. The external recharger unit can also include a recharger control unit 158. The recharger control unit 158 can include a display screen 160. The recharger control unit 158 can also include a first user control button 162 and a second user control button 164.

In various embodiments, the recharger coil 154 of the external recharger unit can be moved within a short distance of the recharge coil 110 of the recharge leads 108. Power can be effectively transferred via an inductive mechanism between the recharger coil 154 and the recharge coil 110. A current flow through the recharger coil 154 (or source coil) produces a magnetic flux which induces a current flow in the recharge coil (or load coil). In various embodiments herein, the recharger coil 154 is positioned within 1, 2, 3, 4, 5, 7, 10, 15, 20, 25, or 30 centimeters from the recharge coil 110. While inductive power transfer is one wireless power transfer technique contemplated herein, it will be appreciated that in some embodiments other techniques of wireless power transfer can also be used including, for example, radiative or other near-field or far-field power transfer techniques power such as transfer through electromagnetic radiation, like radio waves, microwaves or the like.

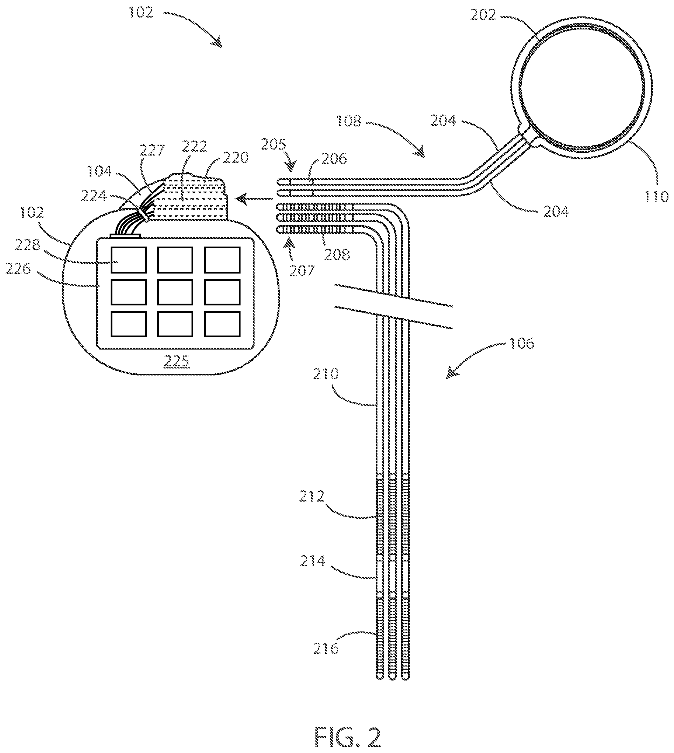

Referring now to FIG. 2, a schematic view of some implantable components 102 of a system is shown in accordance with various embodiments herein. The implantable components 102 can include therapy leads 106. The implantable components 102 also includes an implantable recharge lead 108.

The implantable field generator unit 103 includes a header 104 and a housing 105. The header 104 includes a recharge lead receiving channels 220. The header 104 also includes a therapy lead receiving channels 222. The header 104 also includes a plurality of conductors 227 that can serve to provide electrical communication through the header 104 and between components inside the housing 105 and the conductors within the leads connected to the header.

The housing includes a hermetically sealed interior volume 225. The housing 105 includes a feed-through structure 224 which can provide for the passage of conductors therethrough while also maintaining the hermeticity of the interior volume 225. The implantable electrical field generator unit can include various components within the housing 105 including, for example, a circuit board 226. The circuit board 226 can include electrical field generation circuitry 228 disposed thereon.

Electric field generation circuitry and/or device control circuitry herein can include various components including, but are not limited to, a microprocessor, memory circuit (such as random access memory (RAM) and/or read only memory (ROM)), recorder circuitry, controller circuit, a telemetry circuit, a power supply circuit (such as a battery), a timing circuit, and an application specific integrated circuit (ASIC), a recharging circuit, amongst others.

The therapy leads 106 can include a therapy connection plug 207. In some embodiments the therapy connection plug 207 can be a standard size or configuration. For example, in some embodiments, the therapy connection plug 207 can comply with one or more standards associated with IS-1, DF-1, IS4, and DF4. However, in other embodiments, the therapy connection plug 207 can be configured as a custom size or configuration. The therapy leads 106 can also include therapy connection electrodes 208 that can be associated with the therapy connection plug 207 and can serve to facilitate an electrical interconnection between a contacts or electrodes inside the header and the conductors within the therapy leads 106.

The therapy leads 106 can also include a therapy lead body 210. The therapy connection lead body can extend from the therapy connection plug 207 near the proximal end of the therapy leads 106 down to the therapy electrodes, which are typically closer to the distal end of the therapy leads 106. In various embodiments, the therapy lead body 210 can include a flexible and biocompatible layer on the outside thereof (such as a flexible and biocompatible polymer layer) and can house various electrical conductors and features such as a guidewire lumen on the inside.

In various embodiments, the therapy leads 106 can include at least two coil electrodes disposed thereon. For example, in some embodiments, the therapy leads 106 can include a proximal coil electrode 212 and a distal coil electrode 216. The therapy leads 106 can also include a gap 214 disposed between the proximal coil electrode 212 and the distal coil electrode 216.

The implantable recharge lead(s) 108 can include various components. For example, the implantable recharge lead 108 can include a recharge coil 110, recharge lead bodies 204, and recharge connection plugs 205. The recharge lead body 204 interconnects the connection plug and the recharge coil 110. In the embodiment shown in FIG. 2, there are two recharge lead bodies 204. However, in other embodiments there may only be one or there may be more than two. The recharge lead body 204 can include a flexible and biocompatible layer on the outside thereof (such as a flexible and biocompatible polymer layer) and can house various electrical conductors and features on the inside. In some embodiments, the recharge lead body 204 can also include a guidewire lumen disposed therein. However, in some embodiments, a guidewire lumen may be omitted.

The recharge connection plugs 205 (one for each lead body 204) can include one or more connection electrodes 206. In some embodiments each recharge connection plug 205 can be a standard size or configuration. For example, in some embodiments, the recharge connection plugs 205 can comply with one or more standards associated with connection plugs such as the DF-1 standard (specifying a unipolar connector assembly). Other standards of which one or more parameters can be used include, but are not limited to, IS-1, IS4, DF4 and the like. While FIG. 2 shows the use of two separate connection plugs 205, it will be appreciated that in some embodiments only a single recharge connection plug may be used and in still other embodiments three or more recharge connection plugs may be used. Thus, in some embodiments, the implantable recharge lead(s) 108 can include any number of plugs to be received by the header 104. However, it has been found that the use of at least two recharge connection plugs 205 can be ideal to provide sufficient current flow so that recharging can be performed expeditiously.

The recharge coil 110 can specifically include a conductor coil 202. The conductor coil 202 can be formed of various conductive materials. In some embodiments, the conductor coil can be formed of a coil of wire such as wire made from one or more of copper, aluminum, silver, platinum, nickel, or various metal alloys.

Many different configurations for the arrangement of ports/receiving channels in the header are contemplated herein. The ports/receiving channels can be arranged on top of each other in a stacked configuration, side-by-side, a combination thereof, or any other given configuration. However, in some embodiments, the recharge lead receiving channels 220 (or ports) are specifically disposed on top of the of therapy lead receiving channels 222. While not intending to be bound by theory, this arrangement can be advantageous as the therapy leads may include a greater number of discrete connection electrodes to be connected to and this becomes easier if the receiving channel or port is longer. In general, the receiving channels or ports close to the top of the header include less space for a long receiving channel or port, therefore it can be advantageous for the recharge lead receiving channels 220 (or ports) to be disposed on top (e.g., farther away from the housing) of the therapy lead receiving channel 222.

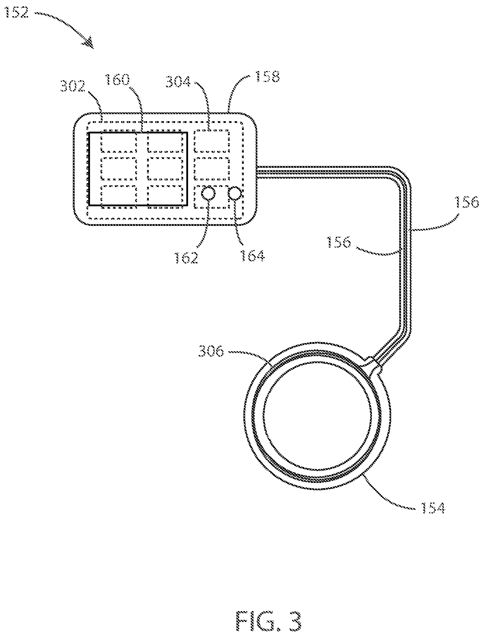

Referring now to FIG. 3, a schematic view of external components 152 of a system are shown in accordance with various embodiments herein. The external components 152 can specifically include an external recharger unit includes a recharger coil 154. The recharger coil 154 can specifically include a recharger conductor coil 306.

The recharger conductor coil 306 can be formed of various conductive materials. For example, in some embodiments, the recharger conductor coil 306 can be formed of a coil of wire such as wire made from one or more of copper, aluminum, silver, platinum, nickel, or various metal alloys.

The external recharger unit can also include a recharger lead 156. The external recharger unit also can also include a recharger control unit 158. The recharger control unit 158 can serve to generate a current flow through the recharger coil 154 so as to produce a magnetic flux.

In some embodiments, the recharger control unit 158 can include a display screen 160. In some embodiments, the recharger control unit 158 can also include a first user control button 162 and in some cases a second user control button 164. Inside, the external recharger unit can include a recharge circuit board 302 along with various recharge circuitry components 304 disposed thereon in order to generate a current flow as well as to store data regarding aspects of the same such as the total charging time, charging history, and the like.

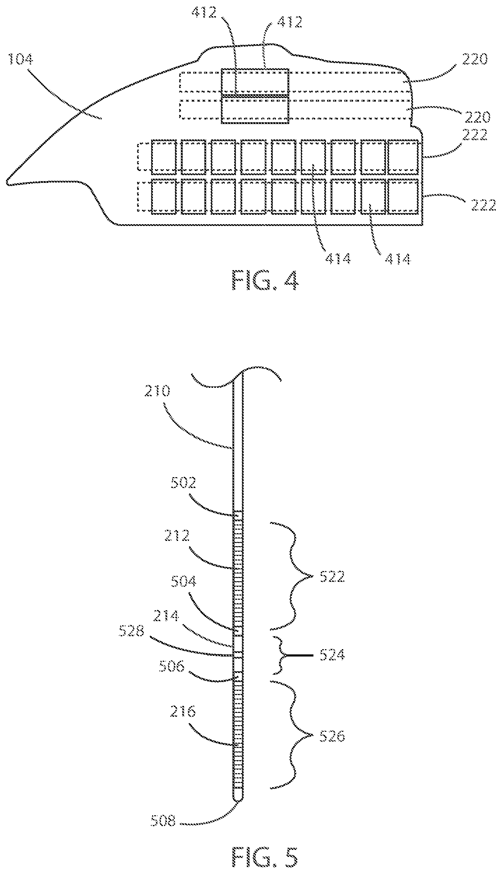

Headers herein can serve to receive connection plugs of leads (therapy, recharge, etc.) and facilitate an electrical connection between the same and components inside the implantable housing (or can). It will be appreciated that headers herein can include various components. Referring now to FIG. 4, a schematic view of a header 104 is shown in accordance with various embodiments herein. The header 104 includes recharge lead receiving channels 220. The header 104 also includes therapy lead receiving channels 222. As described above, these channels or ports can comply with one or more aspects of various connector standards including, but not limited to IS-1, DF-1, IS4, and DF4. In some embodiments herein, the recharge lead receiving channels 220 (or ports) comply with dimensional parameters of the DF-1 standard.

The recharge lead receiving channels 220 can include one or more recharge electrode connector(s) 412. The therapy lead receiving channels 222 can include a plurality of therapy lead connector electrodes 414. The arrangement of receiving channels or ports within the header can vary. However, in various embodiments, the one or more recharge lead receiving channels 220 are disposed on top of the plurality of therapy lead receiving channels 222.

Referring now to FIG. 5, a schematic view of a portion of a therapy lead is shown in accordance with various embodiments herein. In various embodiments, the therapy lead can include at least two coil electrodes. In some embodiments, the system can be configured so that each coil electrode can operate completely independently from one another. However, in other embodiments, the system can be configured so that two coiled electrodes can operate together as a common electrode. In some embodiments, the two coiled electrodes can be configured such that they are arranged in parallel in the circuit to deliver electrical stimulation therapy or can be configured so that they function as if they were in parallel in the circuit. In some embodiments, the two coiled electrodes can be configured such that they are arranged in series in the circuit to deliver electrical stimulation therapy or can be configured so that they function as if they were in series in the circuit. However, in some embodiments, the therapy lead can include a single coil electrode. In some embodiments, the therapy lead can include more than two coil electrodes.

The therapy lead also includes a therapy lead body 210. The therapy lead also includes a first conductor ring 502. The therapy lead also includes a proximal coil electrode 212. The therapy leads also includes a second conductor ring 504. The therapy lead also includes a gap 214. The gap 214 can include a gap length 524. In some embodiments, the therapy lead also includes a temperature sensor 528. In various embodiments, the therapy leads 106 can include a temperature sensor 528. Exemplary temperature sensors herein can include thermistors, thermocouples, resistive temperature detectors, and the like.

The therapy lead also includes a third conductor ring 506. The therapy lead also includes a distal coil electrode 216. The therapy leads also includes a lead tip 508. In some embodiments, the conductor rings can serve as a transition between the portion of the lead body covered with the flexible, biocompatible material (such as a polymer) and the coil electrode. In some embodiments, the conductor rings can serve as a conductive interface between a conductor wire in the lead body and the coil of the coil electrode. For example, in some embodiments, a conductor wire can be soldered, welded or otherwise attached to a conductor ring and the coil of the coil electrode can also be soldered, welded, or otherwise attached to the conductor ring. It will be appreciated that in some embodiments, the lead tip 508 can serve as a conductor. In some embodiments, the lead tip 508 can function like a conductor ring described herein.

The proximal coil electrode 212 can include a proximal coil length 522. In some embodiments, the therapy leads herein can be specialized for use in the treatment of various types of brain cancer. In some embodiments, the proximal coil length 522 of a therapy lead specialized for use in the treatment of various types of brain cancer can be greater than or equal to 0.5 cm, 0.7 cm, 0.9 cm, 1.1 cm, 1.2 cm, 1.4 cm, 1.6 cm, 1.8 cm, or 2.0 cm. In some embodiments, the length can be less than or equal to 6.0 cm, 5.5 cm, 5.0 cm, 4.5 cm, 4.0 cm, 3.5 cm, 3.0 cm, 2.5 cm, or 2.0 cm. In some embodiments, the length can fall within a range from and to any of the foregoing. In some embodiments, the length can fall within a range of 0.5 cm to 6.0 cm, or 0.7 cm to 5.5 cm, or 0.9 cm to 5.0 cm, or 1.1 cm to 4.5 cm, or 1.2 cm to 4.0 cm, or 1.4 cm to 3.5 cm, or 1.6 cm to 3.0 cm, or 1.8 cm to 2.5 cm, or can be about 2.0 cm.

The distal coil electrode 216 can include a distal coil length 526. In some embodiments, the distal coil length 526 of a therapy lead specialized for use in the treatment of various types of brain cancer can be greater than or equal to 0.5 cm, 0.7 cm, 0.9 cm, 1.1 cm, 1.2 cm, 1.4 cm, 1.6 cm, 1.8 cm, or 2.0 cm. In some embodiments, the length can be less than or equal to 6.0 cm, 5.5 cm, 5.0 cm, 4.5 cm, 4.0 cm, 3.5 cm, 3.0 cm, 2.5 cm, or 2.0 cm. In some embodiments, the length can fall within a range from and to any of the foregoing. In some embodiments, the length can fall within a range of 0.5 cm to 6.0 cm, or 0.7 cm to 5.5 cm, or 0.9 cm to 5.0 cm, or 1.1 cm to 4.5 cm, or 1.2 cm to 4.0 cm, or 1.4 cm to 3.5 cm, or 1.6 cm to 3.0 cm, or 1.8 cm to 2.5 cm, or can be about 2.0 cm.

In various embodiments, the proximal coil electrode 212 can include a proximal coil length 522 specialized for use in the treatment of cancer other than brain cancer and/or in other regions of the body beyond the brain. In some embodiments, the proximal coil length 522 specialized for use in the treatment of cancer other than brain cancer can be greater than or equal to 2.0 cm, 2.5 cm, 3.0 cm, 3.5 cm, 4.0 cm, 4.5 cm, 5.0 cm, 5.5 cm, or 6.0 cm. In some embodiments, the length can be less than or equal to 12.0 cm, 10.0 cm, 8.0 cm, 6.0 cm, 5.5 cm, 5.0 cm, 4.5 cm, 4.0 cm, 3.5 cm, 3.0 cm, 2.5 cm, or 2.0 cm. In some embodiments, the length can fall within a range from and to any of the foregoing. In some embodiments, the length can fall within a range of 2.0 cm to 12.0 cm, or 2.5 cm to 8.0 cm, or 3.0 cm to 5.0 cm, or 3.5 cm to 4.5 cm, or can be about 3.5 cm.

In various embodiments, the distal coil electrode 216 can include a distal coil length 526 specialized for use in the treatment of cancer other than brain cancer in other regions of the body. In some embodiments, the distal coil length 526 specialized for use in the treatment of cancer other than brain cancer can be greater than or equal to 2.0 cm, 2.5 cm, 3.0 cm, 3.5 cm, 4.0 cm, 4.5 cm, 5.0 cm, 5.5 cm, or 6.0 cm. In some embodiments, the length can be less than or equal to 12.0 cm, 10.0 cm, 8.0 cm, 6.0 cm, 5.5 cm, 5.0 cm, 4.5 cm, 4.0 cm, 3.5 cm, 3.0 cm, 2.5 cm, or 2.0 cm. In some embodiments, the length can fall within a range from and to any of the foregoing. In some embodiments, the length can fall within a range of 2.0 cm to 12.0 cm, or 2.5 cm to 8.0 cm, or 3.0 cm to 5.0 cm, or 3.5 cm to 4.5 cm, or can be about 3.5 cm.

However, certain other types of cancers may also be desirably treated using coil electrodes with lengths that are similar to those for use with brain cancer.

In embodiments where a single coil electrode is used, the single coil electrode can include a length specialized for use in the treatment of brain cancer or a length specialized for other types of cancer elsewhere in the body. The length of the single coil electrode can be greater than or equal to 2.0 cm, 2.5 cm, 3.0 cm, 3.5 cm, 4.0 cm, 4.5 cm, 5.0 cm, 5.5 cm, 6.0 cm, 6.5 cm, 7.0 cm, 7.5 cm, 8.0 cm, 8.5 cm, 9.0 cm, 9.5 cm, or 10.0 cm. In some embodiments, the length can be less than or equal to 15 cm, 13 cm, 11 cm, 9.5 cm, 9.0 cm, 8.5 cm, 8.0 cm, 7.5 cm, 7.0 cm, 6.5 cm. 6.0 cm, 5.5 cm, 5.0 cm, 4.5 cm, 4.0 cm, 3.5 cm, 3.0 cm, 2.5 cm, or 2.0 cm. In some embodiments, the length can fall within a range from and to any of the foregoing. In some embodiments, the length can fall within a range of 2.0 cm to 15.0 cm, or 3.0 cm to 10.0 cm, or 4.0 cm to 6.0 cm, or can be about 7.0 cm.

In various embodiments, the proximal coil length 522 and the distal coil length 526 are the same. However, in other embodiments, the proximal coil length 522 and the distal coil length 526 are different. In some embodiments, one is larger, but is within approximately 5, 10, 15, 20, 25, 30, 40 or 50 percent of the size of the other. In various embodiments, each of the at least two coil electrodes can be from 0.5 cm to 4 cm in length.

The coil electrodes can have various outside diameters suitable for the treatment of brain cancer. In some embodiments, the outside diameter of the coil electrodes as configured to be desirable for the treatment of brain cancer can be greater than or equal to 0.5 mm, 0.6 mm, 0.7 mm, 0.8 mm, 0.9 mm, 1.0 mm, 1.1 mm, 1.2 mm, or 1.3 mm. In some embodiments, the outside diameter can be less than or equal to 2.0 mm, 1.9 mm, 1.8 mm, 1.7 mm, 1.6 mm, 1.6 mm, 1.5 mm, 1.4 mm, or 1.3 mm. In some embodiments, the outside diameter can fall within a range between any of the foregoing or from 0.5 mm to 2.0 mm, or 0.6 mm to 1.9 mm, or 0.7 mm to 1.8 mm, or 0.8 mm to 1.7 mm, or 0.9 mm to 1.6 mm, or 1.0 mm to 1.6 mm, or 1.1 mm to 1.5 mm, or 1.2 mm to 1.4 mm, or can be about 1.3 mm. In various embodiments, each of the at least two coil electrodes suitable for the treatment of brain cancer has an outside diameter of 1 to 1.5 mm. In various embodiments, each of the at least two coil electrodes suitable for the treatment of brain cancer has an outside diameter of 1.2 to 1.4 mm.

However, the coil electrodes can also have various outside diameters suitable for the treatment of cancers other than brain cancer and/or elsewhere in the body other than the brain. In some embodiments, the outside diameter of the coil electrodes desirable for the treatment of cancers other than brain cancer can be greater than or equal to 2.0 mm, 2.1 mm, 2.2 mm, 2.3 mm, 2.4 mm, 2.5 mm, 2.6 mm, 2.7 mm, 2.8 mm, 2.9 mm, 3.0 mm, 3.1 mm, 3.2 mm, 3.3 mm, 3.4 mm, 3.5 mm, 3.6 mm, 3.7 mm, 3.8 mm, 3.9 mm, 4.0 mm, 4.1 mm, 4.2 mm, 4.3 mm, 4.4 mm, 4.5 mm, 4.6 mm, 4.7 mm, 4.8 mm, 4.9 mm, or 5.0 mm. In some embodiments, the outside diameter of the coil electrodes suitable for the treatment of cancers other than brain cancer can fall within a range between any of the foregoing or from 2.0 mm to 5.0 mm, or 2.2 mm to 5.8 mm, or 2.4 mm to 5.6 mm, or 2.6 mm to 5.4 mm, or 2.8 mm to 5.2 mm, or 3.0 mm to 5.0 mm, or 3.2 mm to 4.8 mm, or 3.4 mm to 4.6 mm. In various embodiments, each of the at least two coil electrodes configured for the treatment of cancers other than brain cancer has an outside diameter of 2.0 to 5.0 mm. In various embodiments, each of the at least two coil electrodes suitable for the treatment of cancers other than brain cancer has an outside diameter of 3.0 to 4.0 mm. However, certain other types of cancers may also be desirably treated using coil electrodes with outside diameters that are similar to those for use with brain cancer.

In various embodiments, the therapy leads 106 can include a gap 214, wherein the gap 214 can be disposed between each of at least two coil electrodes. In some embodiments, the gap 214 length can be greater than or equal to 2.0 mm, 2.6 mm, 3.2 mm, 3.8 mm, 4.4 mm, or 5.0 mm. In some embodiments, the gap length can be less than or equal to 8.0 mm, 7.4 mm, 6.8 mm, 6.2 mm, 5.6 mm, or 5.0 mm. In some embodiments, the gap length can fall within a range between any of the foregoing or from 2.0 mm to 8.0 mm, or 2.6 mm to 7.4 mm, or 3.2 mm to 6.8 mm, or 3.8 mm to 6.2 mm, or 4.4 mm to 5.6 mm, or can be about 5.0 mm. In various embodiments, the gap 214 can be from 4 mm to 6 mm in length.

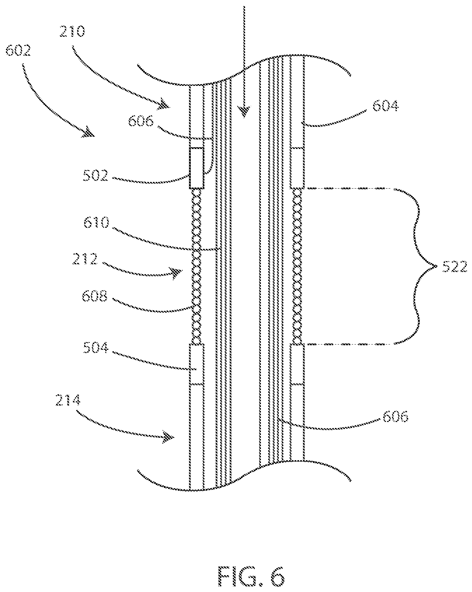

Referring now to FIG. 6, a sectional view of a portion 602 of a therapy lead is shown in accordance with various embodiments herein. FIG. 6 shows a number of components described with respect to FIG. 5 and earlier figures herein. For example, the therapy lead can include a therapy lead body 210, a proximal coil electrode 212 having a proximal coil length 522, a gap 214, a first conductor ring 502, and the like. However, FIG. 6 also shows a flexible outer wall 604 of the lead body along with an electrode conductor 606, which electrically connects to the first conductor ring 502. Thus, the electrode conductor 606 can be in electrical communication with the conductor ring 502, which can in turn be in electrical communication with the coiled conductor 608. However, in some embodiments, the electrode conductor 606 can be directly electrically connected to the coiled conductor 608. Thus, in various embodiments, an electrode conductor can be connected to the coiled conductor 608 directly or indirectly. FIG. 6 also shows conductors 610 passing through this portion of the lead which may connect to other electrodes that are part of the therapy lead or sensors thereon such as a temperature sensor or the like. The coiled conductor 608 can be formed of many different biocompatible and conductive metals or metal alloys including, but not limited to, MP35N, platinum-iridium, silver, platinum, gold, platinum-clad titanium, tantalum-clad titanium, or the like.

In various embodiments, the therapy leads 106 can include a guidewire lumen. The guidewire lumen can facilitate the use of a guidewire such as with an over-the-wire type technique in order to place the therapy leads 106. However, in other embodiments, a guidewire lumen is omitted. In some embodiments, standard tunneling tools can be used to create a tunnel through which the therapy leads 106 then pass.

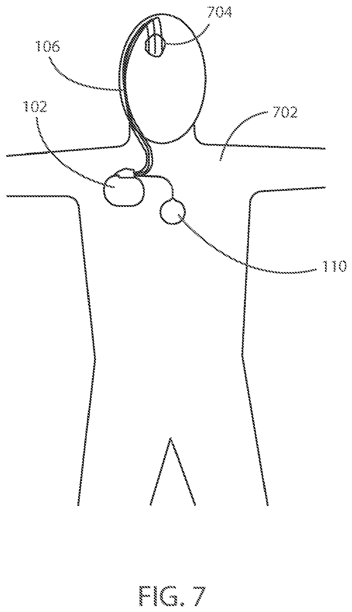

Referring now to FIG. 7, a schematic view of a patient 702 fitted with a system is shown in accordance with various embodiments herein. The electrical stimulation-based cancer therapy system implanted within the patient 702 includes an implantable components 102 such as therapy leads 106, implantable recharge leads 108 including a recharge coil 110. In this view, the patient 702 is shown to include a tumor 704. The therapy leads 106 can be implanted within the patient 702 in order to interface with the tumor 704 and/or be adjacent to the tumor 704 such that electrical fields generated through the therapy leads 106 can interface with the tumor 704.

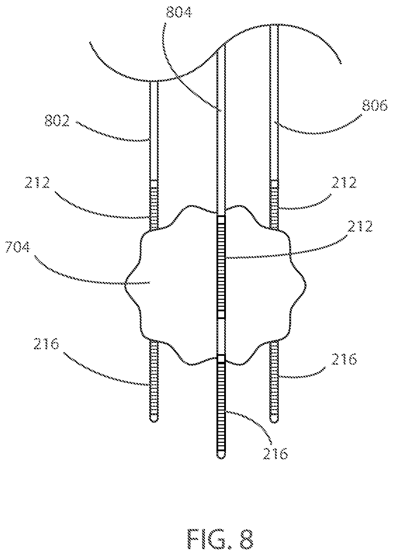

Referring now to FIG. 8, a schematic perspective view of therapy leads 106 interfacing with a tumor 704 is shown in accordance with various embodiments herein. The therapy leads herein include a first therapy lead 802, a second therapy lead 804, and a third therapy lead 806. However, it will be appreciated that a greater or lesser number of therapy leads can be used in other embodiments. The therapy leads in this embodiments all include two coil electrodes 212 (a proximal coil electrode 212 and a distal coil electrode 216). However, it will be appreciated that in some embodiments a greater or lesser number of coil electrodes can be used. Also, in some embodiments, the various therapy leads may include a different number of coil electrodes from one another. The therapy leads are arranged so be in proximity of a tumor 704. In operation, electrical fields can be generated through the coil electrodes along various stimulation vectors (a pathway through the tissue/tumor interconnecting two different electrodes).

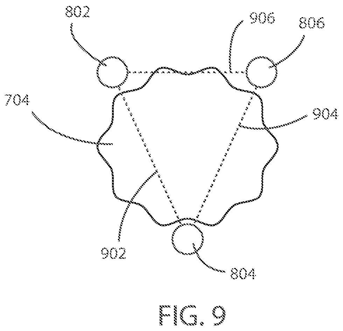

Referring now to FIG. 9, a schematic plan view of therapy leads 106 interfacing with a tumor 704 is shown in accordance with various embodiments herein. Similar to that shown in FIG. 8, therapy leads shown in this view include a first therapy lead 802, a second therapy lead 804, and a third therapy lead 806. This view illustrates a first stimulation vector 902 between at least one electrode on the first therapy lead 802 and at least one electrode on the second therapy lead 804. This view also illustrates a second stimulation vector 904 between at least one electrode on the second therapy lead 804 and at least one electrode on the third therapy lead 806. This view also illustrates a third stimulation vector 906 between at least one electrode on the third therapy lead 806 and at least one electrode on the first therapy lead 802. The stimulation vectors pass through the tumor 704. It will be appreciated that the stimulation vectors can be utilizing one or both of the proximal and distal coil electrodes on the various leads.

In the example of FIGS. 8 and 9, the therapy leads are arranged around the tumor 704 to form a triangle, such as approximately in the form of an equilateral triangle. However, it will be appreciated that the therapy leads can also be arranged in other patterns. In the example of FIGS. 8 and 9, the therapy leads are arranged around the tumor 704 such that the portions bearing the coil electrodes are approximately parallel to one another. However, it will be appreciated that the therapy leads can also be arranged such that the portions bearing the coil electrodes are not parallel to one another.

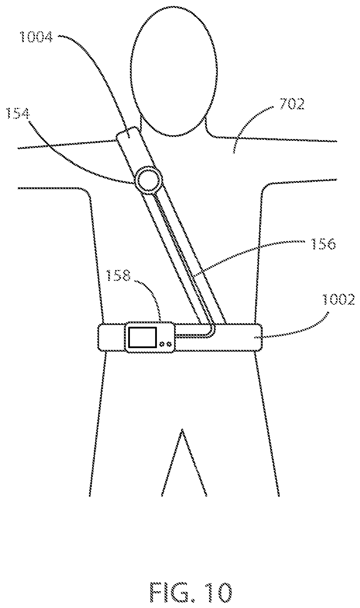

The external components 152 of the system herein can held in place either manually or using a harness or belt-like device. Referring now to FIG. 10, a schematic view of a patient 702 fitted with external components 152 of a system is shown in accordance with various embodiments herein. In this example, the external components include a belt 1002 and a shoulder strap 1004. The belt 1002 and shoulder strap 1004 can hold portions of the external recharger unit. For example, the belt 1002 can hold the recharger control unit 158 and the shoulder strap 1004 can hold the recharger coil 154, with the recharger lead 156 interconnecting the recharger control unit 158 and the recharger coil 154 and passing along both the belt 1002 and the shoulder strap 1004.

### Methods

Many different methods are contemplated herein, including, but not limited to, methods of making, methods of using, and the like. Aspects of system/device operation described elsewhere herein can be performed as operations of one or more methods in accordance with various embodiments herein.

In an embodiment, a method of delivering electrical stimulation therapy to a patient is included, the method can include implanting an implantable electric field generator unit, implanting a plurality of therapy leads, implanting an implantable recharge lead, connecting the plurality of therapy leads to the implantable electric field generator unit, and connecting the implantable recharge lead(s) to the implantable electric field generator unit.

In an embodiment of the method, implanting the therapy leads is performed using a tunneling technique. In an embodiment of the method, implanting the therapy leads is performed using an over-the-wire placement technique.

In an embodiment of the method, the implantable electric field generator unit is implanted separately from the implantable recharge lead.

In an embodiment of the method, implanting the implantable recharge lead is performed using a tunneling technique. In an embodiment of the method, implanting the implantable recharge lead is performed using an over-the-wire placement technique.

In an embodiment of the method, the implantable recharge lead is removably coupled to a header.

In an embodiment, the therapy leads can include a plurality of electrodes. In an embodiment, the electrodes can include at least two coil electrodes. In an embodiment of the method, each of the at least two coil electrodes is from 0.5 cm to 4 cm in length. In an embodiment of the method, each of the at least two coil electrodes has an outside diameter of 1.2 to 1.4 mm.

In an embodiment, the implantable electric field generator unit can include a housing, electrical field generation circuitry disposed within the housing, and a header coupled to the housing. In an embodiment, the header can include a plurality of therapy lead receiving channels or ports and one or more recharge lead receiving channels or ports. In an embodiment of the method, the one or more recharge lead receiving channels are disposed on top of the plurality of therapy lead receiving channels.

In an embodiment, the method can further include fitting the patient with an external recharger unit. In an embodiment, the external recharger unit can include a recharge control unit, a recharge lead, and a recharger coil.

In an embodiment, the method can further include tunneling a path through tissue between the implantable electric field generator unit and the implantable recharge lead, wherein the tunneled path is smaller than the recharger coil.

In an embodiment, the therapy leads can include a gap. In an embodiment of the method, the gap is disposed between each of at least two coil electrodes. In an embodiment of the method, the gap is from 0.5 cm to 3 cm in length.

In an embodiment, the method can further include administering electrical stimulation therapy as generated by the implantable electric field generator unit to the patient.

In an embodiment, the therapy leads can include a temperature sensor. In an embodiment, the temperature sensor can include a thermistor.

### Therapy Parameters

Systems can deliver electrical stimulation therapy that is effective to interfere with the process of mitosis in cancerous cells. Aspects of exemplary therapy parameters are provided as follows. However, it will be appreciated that this is merely provided by way of example and that further variations are contemplated herein.

In some embodiments, the system can be configured to deliver an electric field using one or more frequencies selected from a range of within 10 kHz to 1 MHz. In some embodiments, the system can be configured to deliver an electric field at one or more frequencies selected from a range of within 300 kHz to 500 kHz. In some embodiments, the system can be configured to deliver an electric field at one or more frequencies selected from a range of within 100 kHz to 300 kHz. In some embodiments, the system can be configured to periodically deliver an electric field using one or more frequencies greater than 1 MHz.

In some embodiments, the electric field can be effective in disrupting cellular mitosis in cancerous cells. The electric field can be delivered to the site of a cancerous tumor along more than one vector. In some examples, the electric field can be delivered along at least one vector, including at least one of the lead electrodes. In some embodiments, at least two vectors with spatial diversity between the two vectors can be used. The vectors can be spatially and/or directionally separated (e.g., the vectors can be disposed at an angle with respect to one another) by at least about 10, 20, 30, 40, 50, 60, 70, 80 or 90 degrees.

A desired electric field strength can be achieved by delivering an electric current between two electrodes. The specific current and voltage at which the electric field is delivered can vary and can be adjusted to achieve the desired electric field strength at the site of the tissue to be treated. In some embodiments, the system can be configured to deliver an electric field using currents ranging from 1 mAmp to 1000 mAmp to the site of a cancerous tumor. In some embodiments, the system can be configured to deliver an electric field using currents ranging from 20 mAmp to 500 mAmp to the site of a cancerous tumor. In some embodiments, the system can be configured to deliver an electric field using currents ranging from 30 mAmp to 300 mAmp to the site of a cancerous tumor.

In some embodiments, the system can be configured to deliver an electric field using currents including 1 mAmp, 2 mAmp, 3 mAmp, 4 mAmp, 5 mAmp, 6 mAmp, 7 mAmp, 8 mAmp, 9 mAmp, 10 mAmp, 15 mAmp, 20 mAmp, 25 mAmp, 30 mAmp, 35 mAmp, 40 mAmp, 45 mAmp, 50 mAmp, 60 mAmp, 70 mAmp, 80 mAmp, 90 mAmp, 300 mAmp, 125 mAmp, 150 mAmp, 175 mAmp , 400 mAmp, 225 mAmp, 250 mAmp, 275 mAmp, 300 mAmp, 325 mAmp, 350 mAmp, 375 mAmp, 400 mAmp, 425 mAmp, 450 mAmp, 475 mAmp, 500 mAmp, 525 mAmp, 550 mAmp, 575 mAmp, 600 mAmp, 625 mAmp, 650 mAmp, 675 mAmp, 700 mAmp, 725 mAmp, 750 mAmp, 775 mAmp, 800 mAmp, 825 mAmp, 850 mAmp, 875 mAmp, 900 mAmp, 925 mAmp, 950 mAmp, 975 mAmp, or 1000 mAmp. It will be appreciated that the system can be configured to deliver an electric field at a current falling within a range, wherein any of the forgoing currents can serve as the lower or upper bound of the range, provided that the lower bound of the range is a value less than the upper bound of the range.

In some embodiments, the system can be configured to deliver an electric field using voltages ranging from 1 Vrms to 50 Vrms to the site of a cancerous tumor. In some embodiments, system can be configured to deliver an electric field using voltages ranging from 5 Vrms to 30 Vrms to the site of a cancerous tumor. In some embodiments, the system can be configured to deliver an electric field using voltages ranging from 10 Vrms to 20 Vrms to the site of a cancerous tumor.

In some embodiments, the system can be configured to deliver an electric field using one or more voltages including 1 Vrms, 2 Vrms, 3 Vrms, 4 Vrms, 5 Vrms, 6 Vrms, 7 Vrms, 8 Vrms, 9 Vrms, 10 Vrms, 15 Vrms, 20 Vrms, 25 Vrms, 30 Vrms, 35 Vrms, 40 Vrms, 45 Vrms, or 50 Vrms. It will be appreciated that the system can be configured to deliver an electric field at a voltage falling within a range, wherein any of the forgoing voltages can serve as the lower or upper bound of the range, provided that the lower bound of the range is a value less than the upper bound of the range.

In some embodiments, the system can be configured to deliver an electric field using one or more frequencies including 10 kHz, 20 kHz, 30 kHz, 40 kHz, 50 kHz, 60 kHz, 70 kHz, 80 kHz, 90 kHz, 300 kHz, 125 kHz, 150 kHz, 175 kHz, 400 kHz, 225 kHz, 250 kHz, 275 kHz, 300 kHz, 325 kHz, 350 kHz, 375 kHz, 400 kHz, 425 kHz, 450 kHz, 475 kHz, 500 kHz, 525 kHz, 550 kHz, 575 kHz, 600 kHz, 625 kHz, 650 kHz, 675 kHz, 700 kHz, 725 kHz, 750 kHz, 775 kHz, 800 kHz, 825 kHz, 850 kHz, 875 kHz, 900 kHz, 925 kHz, 950 kHz, 975 kHz, 1 MHz. It will be appreciated that the system can be configured to deliver an electric field using a frequency falling within a range, wherein any of the foregoing frequencies can serve as the upper or lower bound of the range, provided that the upper bound is greater than the lower bound.

In some embodiments, the system can be configured to generate one or more applied electric field strengths selected from a range of within 0.25 V/cm to 1000 V/cm. In some embodiments, the system can be configured to generate one or more applied electric field strengths of greater than 3 V/cm. In some embodiments, the system can be configured to generate one or more applied electric field strengths selected from a range of within 1 V/cm to 10 V/cm. In some embodiments, the system can be configured to generate one or more applied electric field strengths selected from a range of within 3 V/cm to 5 V/cm.

In other embodiments, the system can be configured to deliver one or more applied electric field strengths including 0.25 V/cm, 0.5 V/cm, 0.75 V/cm, 1.0 V/cm, 2.0 V/cm, 3.0 V/cm, 5.0 V/cm, 6.0 V/cm, 7.0 V/cm, 8.0 V/cm, 9.0 V/cm, 10.0 V/cm, 20.0 V/cm, 30.0 V/cm, 40.0 V/cm, 50.0 V/cm, 60.0 V/cm, 70.0 V/cm, 80.0 V/cm, 90.0 V/cm, 300.0 V/cm, 125.0 V/cm, 150.0 V/cm, 175.0 V/cm, 400.0 V/cm, 225.0 V/cm, 250.0 V/cm, 275.0 V/cm, 300.0 V/cm, 325.0 V/cm, 350.0 V/cm, 375.0 V/cm, 400.0 V/cm, 425.0 V/cm, 450.0 V/cm, 475.0 V/cm, 500.0 V/cm, 600.0 V/cm, 700.0 V/cm, 800.0 V/cm, 900.0 V/cm, 1000.0 V/cm. It will be appreciated that the system can generate an electric field having a field strength at a treatment site falling within a range, wherein any of the foregoing field strengths can serve as the upper or lower bound of the range, provided that the upper bound is greater than the lower bound.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains.

As used herein, the recitation of numerical ranges by endpoints shall include all numbers subsumed within that range (e.g., 2 to 8 includes 2.1, 2.8, 5.3, 7, etc.).

The headings shall not be viewed to limit or characterize the invention(s) set out in any claims that may issue from this disclosure. As an example, although the headings refer to a "Field," such claims should not be limited by the language chosen under this heading to describe the so-called technical field. Further, a description of a technology in the "Background" is not an admission that technology is prior art to any invention(s) in this disclosure. Neither is the "Summary" to be considered as a characterization of the invention(s) set forth in issued claims.

The embodiments described herein are not intended to be exhaustive or to limit the invention to the precise forms disclosed in the detailed description Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope of the claims.

## Claims

1. An electrical stimulation-based cancer therapy system (100) comprising:
an implantable electrical field generator unit (103), the implantable electrical field generator unit (103) comprising
a housing (105);
a header (104), wherein the header is coupled to the housing (105), wherein the header (104) comprises one or more recharge lead receiving channels (220) and a plurality of therapy lead receiving channels (222), wherein the one or more recharge lead receiving channels (220) are disposed further away from the housing than the plurality of therapy lead receiving channels (222); and
electrical field generation circuitry (228), wherein the electrical field generation circuitry is disposed within the housing (105);
an implantable recharge lead (108), wherein the implantable recharge lead is removably coupled to the header (104);
a plurality of therapy leads (106), the therapy leads comprising a plurality of electrodes; wherein the plurality of therapy leads (106) are removably coupled to the header (104); and
an external recharger unit.

2. The system (100) of any of claims 1,
the external recharger unit comprising:
a recharger coil (154);
a recharger lead (156); and
a recharger control unit (158); and
the implantable recharge lead (108) comprising:
a connection plug (205);
a recharge coil (110); and
a recharge lead body (204), wherein the recharge lead body (204) interconnects the connection plug (205) and the recharge coil (110).

3. The system (100) of claim 2, wherein the connection plug (205) is configured to fit within the header (104).

4. The system (100) of any of claims 2-3, the recharge lead body (204) comprising a guidewire lumen.

5. The system (100) of any of claims 1-4, the housing (105) comprising:
a feed-through structure (224); and
the housing (105) defining a hermetically sealed interior volume (225);
the header (104) comprising a plurality of conductors, wherein the plurality of conductors pass through the feed-through structure (224).

6. The system (100) of any of claims 1-5, the therapy leads (106) comprising at least two coil electrodes.

7. The system (100) of claim 6, wherein each of the at least two coil electrodes is from 0.5 cm to 4 cm in length.

8. The system (100) of claim 6, wherein each of the at least two coil electrodes has an outside diameter of 1 to 1.5 mm.

9. The system (100) of any of claims 1-8, the therapy leads (106) comprising a gap (214);
wherein the gap (214) is disposed between each of at least two coil electrodes; and
wherein the gap (214) is from 2 mm to 8 mm in length.

10. The system (100) of any of claims 1-9, the therapy leads (106) comprising a guidewire lumen.

11. The system (100) of any of claims 1-10, the therapy leads (106) comprising a temperature sensor (528).

12. The system (100) of any of claims 1-11, the temperature sensor (528) comprising a thermistor.

13. The system (100) of claim 1, the electrical field generation circuitry (228) being configured to generate electric fields having frequencies between 100 kHz and 1 MHz.

## Patentansprüche

1. Auf elektrischer Stimulation basierendes Krebstherapie-System (100), das aufweist:
eine implantierbare elektrische Feldgeneratoreinheit (103), wobei die implantierbare elektrische Feldgeneratoreinheit (103) aufweist
ein Gehäuse (105);
ein Kopfstück (104), wobei das Kopfstück mit dem Gehäuse (105) gekoppelt ist, wobei das Kopfstück (104) einen oder mehrere Ladeleitungs-Aufnahmekanäle (220) und mehrere Therapieleitungs-Aufnahmekanäle (222) aufweist, wobei der eine oder die mehreren Ladeleitungs-Aufnahmekanäle (220) weiter vom Gehäuse entfernt angeordnet sind als die mehreren Therapieleitungs-Aufnahmekanäle (222); und
eine elektrische Felderzeugungsschaltung (228), wobei die elektrische Felderzeugungsschaltung innerhalb des Gehäuses (105) angeordnet ist;
eine implantierbare Ladeleitung (108), wobei die implantierbare Ladeleitung lösbar mit dem Kopfstück (104) gekoppelt ist;
mehrere Therapieleitungen (106), wobei die Therapieleitungen mehrere Elektroden aufweisen; wobei die mehreren Therapieleitungen (106) lösbar mit dem Kopfstück (104) gekoppelt sind; und
eine externe Ladeeinheit.

2. System (100) nach Anspruch 1,
wobei die externe Ladeeinheit aufweist:
eine Ladespule (154);
eine Ladeleitung (156); und
eine Ladesteuereinheit (158); und
wobei die implantierbare Ladeleitung (108) aufweist:
einen Verbindungsstecker (205);
eine Ladespule (110); und
einen Ladeleitungskörper (204), wobei der Ladeleitungskörper (204) den Verbindungsstecker (205) und die Ladespule (110) miteinander verbindet.

3. System (100) nach Anspruch 2, wobei der Verbindungsstecker (205) so konfiguriert ist, dass er in das Kopfstück (104) passt.

4. System (100) nach einem der Ansprüche 2 bis 3, wobei der Ladeleitungskörper (204) ein Führungsdrahtlumen aufweist.

5. System (100) nach einem der Ansprüche 1 bis 4, wobei das Gehäuse (105) aufweist:
eine Durchführungsstruktur (224); und
wobei das Gehäuse (105) einen hermetisch abgeschlossenen Innenraum (225) definiert;
wobei das Kopfstück (104) mehrere Leiter aufweist, wobei sich die mehreren Leiter durch die Durchführungsstruktur (224) erstrecken.

6. System (100) nach einem der Ansprüche 1 bis 5, wobei die Therapieleitungen (106) mindestens zwei Spulenelektroden aufweisen.

7. System (100) nach Anspruch 6, wobei jede der mindestens zwei Spulenelektroden eine Länge von 0,5 cm bis 4 cm aufweist.

8. System (100) nach Anspruch 6, wobei jede der mindestens zwei Spulenelektroden einen Außendurchmesser von 1 bis 1,5 mm aufweist.

9. System (100) nach einem der Ansprüche 1 bis 8, wobei die Therapieleitungen (106) einen Spalt (214) aufweisen;
wobei der Spalt (214) zwischen jeder der mindestens zwei Spulenelektroden angeordnet ist; und
wobei der Spalt (214) eine Länge von 2 mm bis 8 mm aufweist.

10. System (100) nach einem der Ansprüche 1 bis 9, wobei die Therapieleitungen (106) ein Führungsdrahtlumen aufweisen.

11. System (100) nach einem der Ansprüche 1 bis 10, wobei die Therapieleitungen (106) einen Temperatursensor (528) aufweisen.

12. System (100) nach einem der Ansprüche 1 bis 11, wobei der Temperatursensor (528) einen Thermistor aufweist.

13. System (100) nach Anspruch 1, wobei die elektrische Felderzeugungsschaltung (228) konfiguriert ist, elektrische Felder mit Frequenzen zwischen 100 kHz und 1 MHz zu erzeugen.

## Revendications

1. Système de thérapie anticancéreuse par stimulation électrique (100), comprenant :
une unité génératrice de champ électrique implantable (103), ladite unité génératrice de champ électrique implantable (103) comprenant
un boîtier (105) ;
un collecteur (104), ledit collecteur étant raccordé au boîtier (105), ledit collecteur (104) comprenant un ou plusieurs canaux de réception de fils de recharge (220) et une pluralité de canaux de réception de fils de traitement (222), le ou les canaux de réception de fils de recharge (220) étant disposés plus loin du boîtier que la pluralité de canaux de réception de fils de traitement (222) ; et
un circuit de génération de champ électrique (228), ledit circuit de génération de champ électrique étant disposé à l'intérieur du boîtier (105) ;
un fil de recharge implantable (108), ledit fil de recharge implantable étant raccordé de manière amovible au collecteur (104) ;
une pluralité de fils de traitement (106), lesdits fils de traitement comprenant une pluralité d'électrodes ; la pluralité de fils de traitement (106) étant raccordée au collecteur (104) de manière amovible ; et
une unité de recharge extérieure.

2. Système (100) selon la revendication 1,
où l'unité de recharge extérieure comprend :
une bobine de recharge (154) ;
un fil de recharge (156) ; et
une unité de commande de recharge (158) ; et
où le fil de recharge implantable (108) comprend :
une fiche de connexion (205) ;
une bobine de recharge (110) ; et
un corps de fil de recharge (204), ledit corps de fil de recharge (204) raccordant la fiche de connexion (205) et la bobine de recharge (110).

3. Système (100) selon la revendication 2, où la fiche de connexion (205) est prévue pour être insérée dans le collecteur (104).

4. Système (100) selon la revendication 2 ou la revendication 3, où le corps de fil de recharge (204) comprend une lumière pour fil-guide.

5. Système (100) selon l'une des revendications 1 à 4, où le boîtier (105) comprend :
une structure traversante d'alimentation (224) ; et
où le boîtier (105) définit un volume intérieur hermétiquement clos (225) ;
le collecteur (104) comprenant une pluralité de conducteurs, la pluralité de conducteurs traversant la structure traversante d'alimentation (224).

6. Système (100) selon l'une des revendications 1 à 5, où les fils de traitement (106) comprennent au moins deux électrodes enroulées.

7. Système (100) selon la revendication 6, où chacune des au moins deux électrodes enroulées a une longueur comprise entre 0,5 cm et 4 cm.

8. Système (100) selon la revendication 6, où chacune des au moins deux électrodes enroulées a un diamètre extérieur compris entre 1 et 1,5 mm.

9. Système (100) selon l'une des revendications 1 à 8, où les fils de traitement (106) comprennent un espacement (214) ;
où l'espacement (214) est disposé entre chacune des au moins deux électrodes enroulées ; et
où l'espacement (214) a une longueur comprise entre 2 mm et 8 mm.

10. Système (100) selon l'une des revendications 1 à 9, où les fils de traitement (106) comprennent une lumière pour fil-guide.

11. Système (100) selon l'une des revendications 1 à 10, où les fils de traitement (106) comprennent un capteur de température (528).

12. Système (100) selon l'une des revendications 1 à 11, où le capteur de température (528) comprend une thermistance.

13. Système (100) selon la revendication 1, où le circuit de génération de champ électrique (228) est prévu pour générer des champs électriques ayant des fréquences comprises entre 100 kHz et 1 MHz.
